# EUROPEAN PATENT SPECIFICATION

(11) **EP 4 165 615 B1**
(45) Date of publication and mention of the grant of the patent: **19.11.2025**
(21) Application number: 21733904.3
(22) Date of filing: 11.06.2021
(51) Int. Cl.: G08B 21/24, G08B 21/22

(54) **HYGIENE COMPLIANCE MONITORING**
HYGIENEKONFORMITÄTSÜBERWACHUNG
SURVEILLANCE DE LA CONFORMITÉ DE L'HYGIÈNE

(30) Priority: 11.06.2020 SE 2050701
(43) Date of publication of application: 19.04.2023
(73) Proprietor: Saaz Engineering AB, 22222 Lund (SE)
(72) Inventor: SOLÉN, Staffan, 22460 Lund (SE)
(74) Representative: Neij & Lindberg AB
(86) International application number: PCT/EP2021/065772
(87) International publication number: WO 2021/250238

(56) References cited:
- WO-A1-2010/099488
- US-A1- 2018 372 500

## Description

### Technical Field

The present disclosure generally relates to hygiene compliance monitoring, and in particular to the use of wearable devices for detecting non-compliance with basic hygiene standards or routines, for example in a healthcare facility or a nursing home.

### Background Art

Hand hygiene is an important aspect of daily life in the fight to prevent or otherwise minimize the spreading of disease. By cleaning one's hands, pathogens, including bacteria and viruses, as well as harmful chemicals and other potentially dangerous substances may be removed, reducing health risks. Soaps, detergents, and alcohol-based hand hygiene agents, such as hand sanitizers, are typically used. Hand hygiene practices are especially important in the administration of medicine and medical care, in the food processing and preparation industries, as well as in preparing and serving food to consumers.

Continuous training and development for both management and staff helps to ensure that high standards of hygiene are maintained alongside operational efficiency. However, in situations of time pressure and stress, it is easy to forget to take all necessary measures to maintain hygiene compliance. It may also be difficult, especially for new staff, to remember and adhere to all routines.

WO 2010/099488 A1 discloses a wearable device for monitoring hygiene compliance according to the preamble of appended independent claim 1.

US 2018/0372500 A1 discloses solutions for determining indoor walking path.

US10002518 discloses a distributed monitoring system comprising badges carried by medical staff, and IR transmitters located at wash stations and in rooms of a medical facility to transmit first and second signals, respectively. The badge receives a first signal when proximate to a wash station. A first indicator on the badge indicates that the wearer has visited the wash station. The badge receives a second signal when inside a room. A second indicator on the badge alerts the wearer of failure to visit the wash station before the room visit. If the first/second signal is received by more than one badge, the system will generate false alerts. Frequent false alerts would defeat the purpose of the system. US10002518 proposes that the IR transmitters at wash stations should have a short signal range, for example 3 feet, and be located directly above the wash station to transmit the first signal in a narrow vertical cone onto the wash station. While such a well-controlled installation may reduce the risk for false alerts, it involves careful calibration and testing of the system before, during and after installation to ensure that the IR transmitters are properly oriented and have a proper signal range. Further, false alerts may still occur if the wash stations are placed in narrow spaces or busy environments.

A corresponding but centralized system is disclosed in US9047755. Here, the staff wears RFID tags and monitoring devices are installed in rooms and at hygiene stations. The monitoring devices comprises an RFID transceiver with a signal range of one or two feet for interacting with the RFID tags and are connected to a central processor. The monitoring devices may detect a visit by a tag at a wash station or a visit by a tag in a room. The central processor may gather information from the monitoring devices and cause a specific monitoring device to generate a warning when the tag of a potentially contaminated wearer is detected in a room. The short signal range of this system limits both its use and its installation. For example, staff may be required to perform special actions to bring the RFID tag within range of the monitoring device and/or the placement of the monitoring devices may be restricted by the range.

Some desirable characteristics of a hygiene compliance monitoring system include that it is able to determine if a user has complied with a hygiene protocol, capable of recording and/or alerting a user that violates the hygiene protocol, simple to deploy, scalable, reliable with low risk of false determination of protocol violation, and unobtrusive by not interfering the ordinary work protocol.

### Summary

It is an objective to at least partly overcome one or more limitations of the prior art.

One objective is to at least partly achieve one or more of the above-mentioned desirable characteristics.

One or more of these objectives, as well as further objectives that may appear from the description below, are at least partly achieved by a wearable device, a system, a method of operating a wearable device, and a computer-readable medium according to the independent claims, embodiments thereof being defined by the dependent claims.

Aspects of the present disclosure involve a wearable device which is configured to determine a compliance status of its wearer, upon receiving a first wireless signal from a hygiene station transponder that is activated by a user of a hygiene station. Such an "intelligent" wearable device enables distributed monitoring of hygiene compliance, which facilitates deployment and provides scalability.

The wearable device is configured to determine first association data that defines a spatial and/or temporal relation between the wearable device and the hygiene station transponder, and to use the first association data to determine the hygiene compliance status of the wearer. The first association data enables the wearable device to discriminate between an activation of the hygiene station transponder by the wearer and an activation by another user. This discrimination significantly relaxes the constraints on the positioning, range and transmission pattern of the hygiene station transponder, since the wearable device is capable of determining if an intercepted first wireless signal is likely or unlikely to be activated by the wearer.

The wearable device is configured to determine the first association data as a function of an output signal of a movement sensor in the wearable device, so that the first association data is at least representative of a movement pattern that corresponds to a walking pattern of the wearer. The movement pattern provides robust, reliable and comprehensible input data for inferring presence or non-presence of the wearer at the hygiene station. Further, the use of a movement pattern allows the wearable device to monitor hygiene compliance independent of external measurement data. Such stand-alone operation of the wearable device facilitates deployment.

The wearable device is configured to set the hygiene compliance status of the wearer as a function of the first association data, including the movement pattern. By determining the hygiene compliance status of the wearer, the wearable device is capable of recording violations of a hygiene protocol and/or to alert the wearer of such violations, for example by activating a feedback device in the wearable device or an external feedback unit.

The determination and use of the first association data also enable the monitoring by the wearable device to be adapted to the hygiene protocol applied in a facility without imposing any changes to the ordinary work protocol at the facility. For example, the monitoring may be performed without requiring staff to stand in certain areas when performing a washing operation at the hygiene station or restricting access to the hygiene stations by staff not performing a washing operation.

Still other objectives, advantages and technical effects, as well as features, embodiments and aspects, will appear from the following detailed description and the drawings.

### Brief Description of the Drawings

Embodiments will now be described, by way of example, with reference to the accompanying schematic drawings.
FIG. 1 is a top plan view of an example installation of a hygiene compliance monitoring system in a health care environment.
FIGS 2A-2B are block diagrams of an example wearable device and an example transponder, respectively, for use in a hygiene compliance monitoring system.
FIG. 3 is a side view of a transponder installed on an example dispenser.
FIG. 4 is a flow chart of an example method of determining a compliance status of a wearer of a wearable device.
FIGS 5A-5G are top plan views for exemplifying various techniques of inferring presence or non-presence of a user at a hygiene station with an activated transponder.
FIG. 6 is a flow chart of an example method of detecting entry of a user into a target zone and alerting the user if found to violate hygiene protocol.
FIGS 7A-7B are flow charts of example methods that give a user time to adhere to hygiene protocol after entry into the target zone.
FIG. 8 is a flow chart of an example method of operating a transponder.
FIG. 9 is a flowchart of a variant of the method in FIG. 6.

### Detailed Description

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements.

Also, it will be understood that, where possible, any of the advantages, features, functions, devices, and/or operational aspects of any of the embodiments described and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention. As used herein, the term "and/or" comprises any and all combinations of one or more of the associated listed elements.

It will furthermore be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing the scope of the present disclosure.

Well-known functions or constructions may not be described in detail for brevity and/or clarity. Unless otherwise defined, all terms (including technical and scientific terms) used herein have the same meaning as commonly understood by one of ordinary skill in the art to which this disclosure belongs.

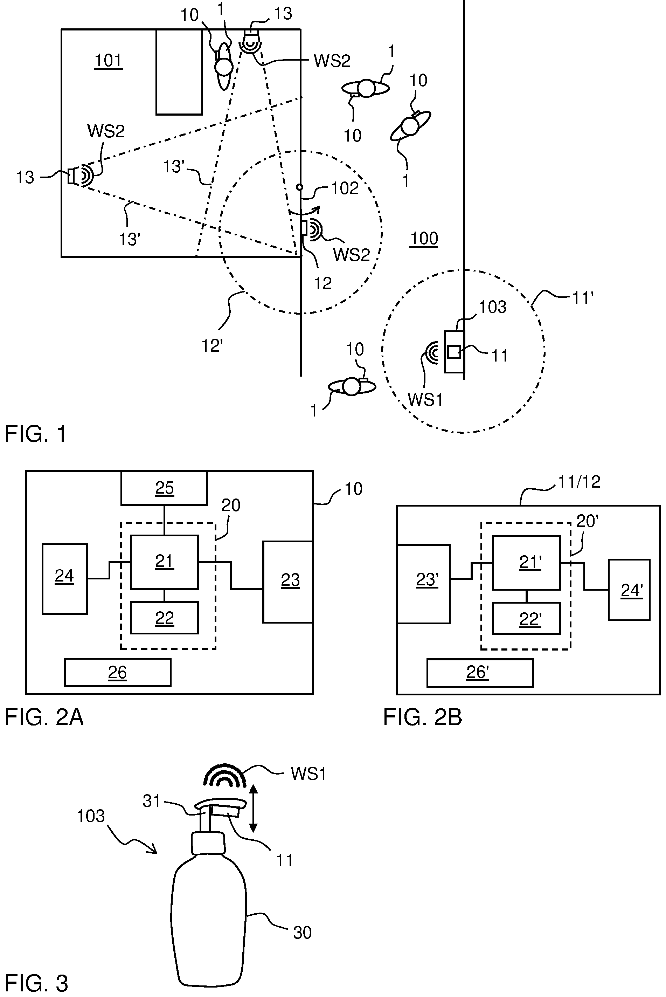

In the following, embodiments will be presented with reference to a healthcare facility shown in top plan view in FIG. 1. The facility may be a hospital or a nursing home and includes a corridor 100 and an adjoining room 101. In the illustrated example, a door 102 physically separates the room 101 from the corridor 100. Thus, the door 102 is an entry point to the room 101. In the following, it is assumed that the room 101 is subject to a hygiene protocol, which requires staff to perform a general or specific washing operation. For example, the room 101 may a sickroom, an isolation room, an ICU room, a surgery, a ward, a nursing room, etc. The washing operation may be performed on a body part and/or on a piece of equipment carried by the staff. In the following, it is assumed that the washing operation is hand washing.

The washing operation is performed at hygiene stations 103 (one shown). In the illustrated example, a hygiene station 103 is located in the corridor 100. In another example, not shown, a hygiene station 103 may be located inside the room 101. The hygiene station 103 may include one or more dispensers of a cleaning fluid such as soap, detergent, disinfectant, sanitizer, etc. The hygiene station 103 may also include a wash basin and a water tap.

FIG. 1 also shows individuals 1 working in the facility. Each individual 1 carries a wearable device 10, denoted "badge" in the following. The badge 10 comprises logic configured to assign a current compliance status to the wearer. In the following examples, the compliance status may be either "compliant" or "non-compliant". In some embodiments, a "compliant" status may indicate that the wearer has performed a washing operation within a given time frame, for example within the last 1-10 minutes. Outside this time frame, the wearer may be assigned the "non-compliant" status, which is thus a default status. In other embodiments, the "compliant" status may only be assigned if further conditions are fulfilled, such as that the washing operation has been performed for a minimum duration, has been performed at a specific type of hygiene station, etc.

In the example of FIG. 1, at least one signaling device or transponder 11 is installed at each hygiene station 103 (one shown). The transponder 11 is configured to be activated to transmit a first wireless signal, WS1, when a user performs a washing operation at the hygiene station 103. Thus, the transponder 11 is configured to sense user activity and is denoted "hygiene station transponder" or HST in the following. In some embodiments, HST 11 is configured and arranged to be activated to transmit WS1 by sensing an action that is inherent to the washing operation, i.e. without requiring a deliberate action by the user. FIG. 1 illustrates, by example, a signal range 11' of the HST 11. In the illustrated example, the signal range 11' is omni-directional and spans a significant part of the corridor 100.

In the example of FIG. 1, a signaling device or transponder 12 is installed on the door 102 at the entry point to the room 101. The transponder 12 is configured to be activated to transmit a second wireless signal, WS2, when the door 102 is opened. Thus, the transponder 12 is configured to sense user activity and is denoted "entry point transponder" or EPT in the following. In some embodiments, EPT 12 is configured and arranged to be activated to transmit WS2 by sensing an action that is inherent to opening the door, i.e. without requiring a deliberate action by the user. FIG. 1 illustrates, by example, a signal range 12' of EPT 12. In the illustrated example, the signal range 12' is omni-directional and spans a significant part of the corridor 100.

As an alternative or supplement to EPT 12, one or more transponders may be installed inside the room 101 to transmit WS2. In the example of FIG. 1, two such room transponders 13, denoted RT, are fixedly arranged to transmit WS2 at least in the direction of the entry point 102. FIG. 1 illustrates, by example, a transmission pattern 13' of the respective RT 13. It should be understood that WS2 may be transmitted omnidirectionally instead of directionally as shown. In some embodiments, RT 13 is configured to continuously transmit WS2. Such continuous transmission may be power-consuming and require RT 13 to be connected to mains power, which may complicate installation. In other embodiments, to reduce power consumption, RT 13 may be selectively activated to transmit WS2 when a presence sensor indicates presence of an individual, for example within the region of the transmission pattern 13'. The presence sensor may be of any known configuration and may, for example, detect presence based on temperature, reflected acoustic or electromagnetic waves, etc. In some embodiments, the presence sensor is integrated in RT 13. In other embodiments, the presence sensor is a separate device. Such a separate device may be a photocell at the entry point 102, or an inertial sensor arranged on the door 102. It is also conceivable to use EPT 12 as a presence sensor for RT 13.

The structure and operation of a hygiene compliance monitoring system in accordance with various embodiments will be described in more detail in the following. As understood from FIG. 1, the monitoring system may comprise wearable devices (badges 10) which are worn by users moving around within the facility, first signaling devices or transponders (HSTs 11) which are installed at one or more hygiene stations 103 within the facility to transmit first wireless signals (WS1), and second signaling devices or transponders (EPTs 12 and/or RTs 13) which are installed at or in rooms to transmit second wireless signals (WS2).

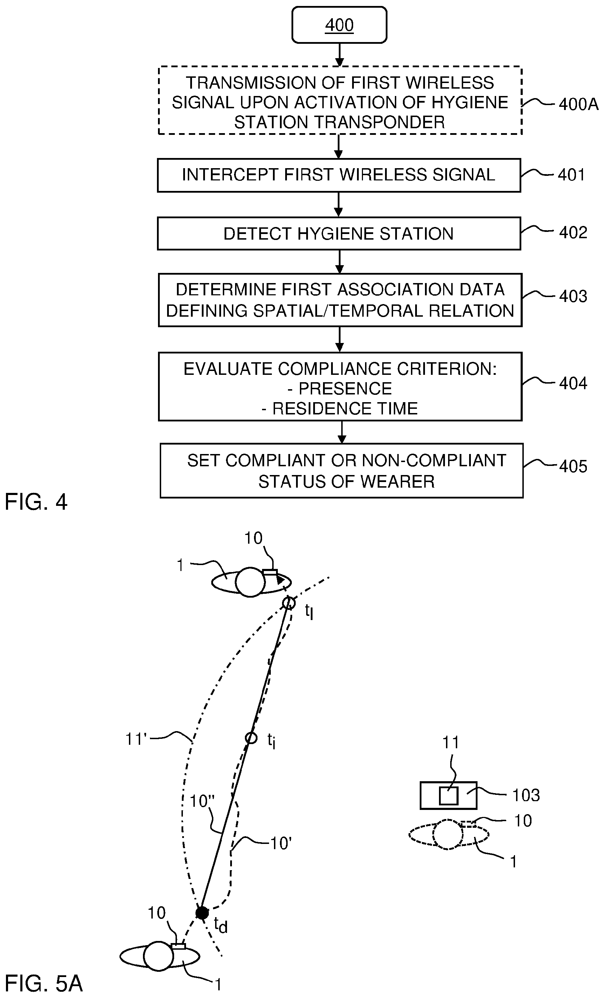

FIG. 2A is a block diagram of an example badge 10. The badge 10 comprises a control system 20 which forms logic for controlling the operation of the badge 10, which will be described further below with reference to FIGS 4-7.

The control system 20 may be implemented by hardware or a combination of hardware and software. In some embodiments, the control system 20 comprises a processing device 21 for executing instructions stored in a memory 22, which may comprise one or more of a buffer, a flash memory, a hard drive, a removable media, a volatile memory, a non-volatile memory, a random access memory (RAM), or another suitable data storage device. The processing device 21 may comprise a central processing unit (CPU), graphics processing unit (GPU), microcontroller, microprocessor, ASIC, FPGA, or any other specific or general processing device. The instructions may be supplied to the memory 22 on a computer-readable medium, which may be a tangible (non-transitory) product (for example magnetic medium, optical disk, read-only memory, flash memory, etc.) or a propagating signal.

The control system 20 is connected to receive signals from a wireless receiver 23 and a movement sensor 24, and to operate a feedback device 25. The badge 10 is powered by a stand-alone power source 26, such as a battery or fuel cell.

The wireless receiver 23 may be configured to receive wireless signals in accordance with a radio access technology (RAT), for example Bluetooth or BLE, RFID, based on IEEE 802.15.4 such as Zigbee, Thread, ISA100.11a or Wireless HART, based on IEEE 802.11 such as WiFi, based on IEEE 802.16 such as WiMAX, or by cellular communication, for example as proposed for loT devices. In an alternative, the receiver 23 is configured for IR (infrared) communication. In some embodiments, the receiver 23 is configured for short-range wireless communication and has a signal range of less than 200, 100, 50, 10, 5 or 1 meter.

The movement sensor 24 is configured to sense the motion of the badge 10. In some embodiments, the movement sensor 24 is a self-contained device, which is configured to generate an output signal indicative of one or more of relative positions, speed or acceleration of the badge 10. The term "self-contained" is intended to imply that the movement sensor 24 is a stand-alone device capable of generating the output signal without input from external devices. For example, the movement sensor 24 may comprise one or more accelerometers, gyroscopes, magnetometers, strain gauges, force sensors, etc. In some embodiments, the movement sensor 24 comprises an inertial sensor, for example an IMU.

The feedback device or indicator 25 is operable to generate a feedback signal to notify or alert the wearer of the badge 10. The feedback signal may visual, audible, tactile, etc. In some embodiments, the feedback device 25 comprises one or more of a lamp, LED, vibrator, speaker, beeper, etc.

It should be understood that the badge 10 may comprise further components not shown in FIG. 2A, such as a wireless transmitter, one or more control buttons, one or more ports for connecting external devices and/or charging the power source 26, a timer, etc. Further, one or more components may be merged into a physical unit. For example, the receiver 23 may be merged with the control unit 20.

FIG. 2B is a block diagram of an example transponder, which may correspond to the above-mentioned HST 11 or EPT 12. A control system 20' is configured and arranged to provide signals to a wireless transmitter 23' and to receive signals from a movement sensor 24'. The transmitter 23' is configured to generate and broadcast wireless signals for receipt by the receiver 23 in a badge 10 located within signal range. The transponder 11/12 is powered by a stand-alone power source 26'. The components of the transponder 11/12 may be identical or similar to corresponding components of the badge 10 and will not be described in further detail. Like for the badge 10, the transponder 11/12 may comprise further components not shown in FIG. 2B, such as a wireless receiver, one or more control buttons, one or more ports for connecting external devices and/or charging the power source 26', a timer, etc. Further, one or more components may be merged into a physical unit. For example, the transmitter 23' may be merged with the control unit 20'.

In some embodiments, the transponder 11/12 is configured to include a transponder identifier, TID, in the wireless signal. The TID may identify the transponder 11/12 to the badge 10 that intercepts the wireless signal. In one example, the TID may differ between HSTs 11 and EPTs 12 and thus differentiate WS1 and WS2. In another example, the TID may differ between different types of hygiene stations 103 and/or different types of entry points 102. In yet another example, the TID may differ between different types of cleaning equipment at a hygiene station 103, for example different types of cleaning fluid.

The transponder 11/12 is configured to transmit a wireless signal whenever the output signal of the movement sensor 24' is indicative of a predefined motion, as determined by the control unit 20'. The predefined motion may be generic or specific. In one example, the transponder 11/12 may triggered by any motion, optionally provided that the motion has a minimum duration. In another example, the transponder 11/12 may triggered by a specific movement pattern, such as a back and forward motion corresponding to a pumping action or a swinging or pivoting motion corresponding to the opening of a door.

FIG. 3 shows an example installation of HST 11 in a hygiene station 103, specifically in a dispenser of cleaning fluid. The dispenser comprises a container 30 for the cleaning fluid and a pumping mechanism 31. A user may operate the pumping mechanism 31 back and forth, as shown by an arrow, to draw the cleaning fluid from the container for discharge through a pump spigot or the like. As shown, HST 11 responds to the user operation by transmitting WS1.

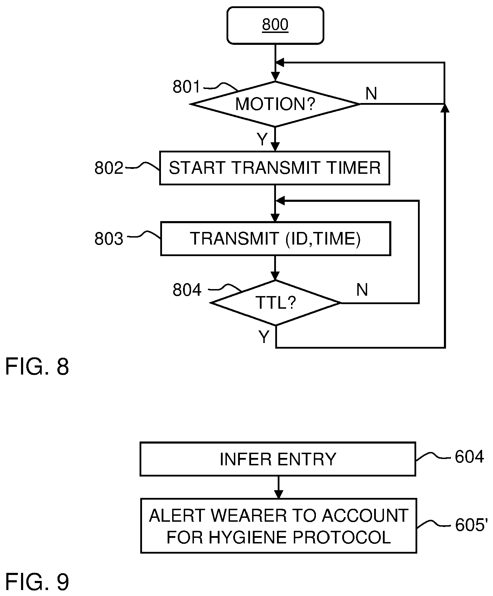

FIG. 8 is a flow chart of an example method 800 for operating a transponder, for example the transponder 11/12 in FIG. 2B. In step 801, the output signal of the movement sensor 24' is evaluated for detection of the above-mentioned predefined motion. When such motion is detected, the method proceeds to step 802, which starts a timer to generate timer values in a monotonically increasing or decreasing sequence of values. The timer values thereby directly or indirectly represent a time from motion detection by step 801. In step 803, a wireless signal is transmitted to include the TID and a current timer value. For HSTs 11, the wireless signal is WS1. For EPTs 12, the wireless signal is WS2. The signals WS1 and WS2 are at least distinguishable by TID. Step 803 is performed repeatedly until a transmission time limit, TTL, is reached, as detected by step 804, which returns the method to step 801. Thereby, the transmission of the wireless signal is terminated and the transponder 11/12 is set to wait for detection of the predefined motion. The method 800 may operate with any suitable time interval between the transmissions in step 803. In some embodiments, the time interval is randomized to reduce the risk of collisions between wireless signals from different transponders 11/12.

In one implementation example, the transmitter 23' is a BLE transmitter, which is configured to implement step 803 by broadcasting data packets using BLE advertisements with a suitable advertisement interval. The transponder 11/12 is thereby operated as a motion-triggered BLE beacon.

The transponder 11/12 may be configured to be in a low-power mode ("sleep mode") by default and enter an active mode upon detection of a dedicated wake-up event, for example when the movement sensor 24' signals a motion. The transponder 11/12 may return to the low-power mode in the absence of motion, optionally after a configurable time delay.

The foregoing description of the transponder 11/12 is also applicable to RTs 13. However, since RTs 13 are typically fixed, the movement sensor 24' may be omitted and, optionally, replaced by a presence sensor of any known configuration. Correspondingly, step 801 of the method 800 in FIG. 8 may be modified to evaluate an output signal of the presence detector for detection of presence of a user.

The monitoring system in FIG. 1 is decentralized, and logic in the badges 10 is configured to implement the monitoring, for example as shown in FIG. 4. The method 400 presumes a step 400A, which is performed by HST 11 at a hygiene station 103 and involves transmission of WS1. The transmission of WS1 is indicative of an initiated and possibly ongoing washing operation at the hygiene station 103. In step 401, the receiver 23 of the badge 10 intercepts WS1, if the receiver 23 is within range of the transmitter 23' of HST 11. Subsequent steps 402-405 are performed by the processing system 20 of the badge 10. In step 402, the hygiene station is detected based on WS1. Step 402 may comprise determining that the intercepted signal is a WS1 and thus originates from an HST 11. Optionally, step 402 may also determine, based on the TID or supplemental data in WS1, that WS1 originates from a specific type of hygiene station, a specific hygiene station, or even a specific component of a hygiene station, such as a specific container of cleaning fluid, a water tap, etc. In step 403, first association data is determined based on WS1 and/or the output signal from the movement sensor 24 in the badge 10. The first association data defines a spatial and/or temporal relation between the badge 10 and the HST 11 that has transmitted WS1. The determination of the first association data by step 403 goes beyond just detecting presence of WS1, which is done by step 402, and comprises extracting, deriving or computing one or more spatial and/or temporal parameter values from WS1 and/or the output signal of the movement sensor 24. The first association data from step 403 is then used for determining the current compliance status and setting a status indicator to "compliant" or "non-compliant" for the wearer of the badge 10.

The first association data addresses the problem of determining if HST 11 has been activated by the wearer of the badge 10 or by another user, given that more than one badge 10 may be capable of intercepting WS1 transmitted by HST 11, as understood from FIG. 1. In the embodiment shown in FIG. 4, the method 400 comprises a step 404 which evaluates a compliance criterion, and a step 405 that sets the status indicator to "compliant" if the compliance criterion is fulfilled in step 404. In some embodiments, the "compliant" status is only valid for a limited time. Thus, step 405 may start a timer when setting the status indicator to "compliant", and change the status indicator to "non-compliant" when the timer reaches a time-out value. If the compliance criterion is not fulfilled in step 404, step 405 may set the status indicator to "non-compliant", unless the status indicator is set to "non-compliant" by default.

The compliance criterion comprises one or more rules ("presence rule") for determining, based on the first association data, if the wearer of the badge 10 is likely to have been present at HST 11. In some embodiments, the compliance criterion may also comprise one or more rules ("completion rule") for determining if the wearer is likely to have completed the washing operation at the hygiene station, for example based on an estimated residence time of the wearer at the hygiene station.

FIGS 5A-5G illustrate some spatial and/or temporal parameters that may be included in the first association data, and corresponding rules for determining or inferring presence. FIGS 5A-5G will be described with reference to a badge 10 that is worn by an individual 1 represented by solid lines. In some examples, this individual is not present at the hygiene station 103 and has thus not activated the HST 11. The badge 10 of the solid-lined individual 1 is capable of inferring a presence or lack of presence by analysis of a respective parameter as described in the following.

In some embodiments, the first association data is representative of a movement pattern of the badge 10 during a time period. The movement pattern corresponds to the walking pattern of the individual 1 and may be estimated based on the output signal of the movement sensor 24 (FIG. 2A). As used herein, the walking pattern designates the path of the wearer as the wearer moves within a facility, for example as seen in a top plan view (cf. FIG. 1). The path may be defined in a two- or three-dimensional coordinate system and may include temporal information, which defines the dynamics or "intensity" of the path, such as speed, or acceleration. In some embodiments, the movement pattern comprises data on one or more of displacement, speed or direction between consecutive time points. For example, the movement pattern may be represented as a time sequence of relative positions. The determination of the movement pattern based on measurement data from a movement sensor 24 is well-known as such. For example, accelerometer data may be processed for determination of one or more of acceleration, speed, and displacement. Gyrometer data may be additionally processed to determine direction of movement, and magnetometer data may be processed to quantify the direction in relation to a cardinal direction. The movement pattern will inherently be given in relation to HST 11, which is effectively stationary. However, the location of HST 11 is not known to the badge 10 from the movement pattern.

In some embodiments, a compliance criterion based on movement pattern may generally comprise determining one or more pattern-related parameter values representative of the movement pattern and evaluating presence based on the pattern-related parameter value(s).

FIG. 5A illustrates a use case in which the HST 11 is activated by another individual 1 (dashed lines), and the badge 10 of the wearer 1 (solid lines) detects and evaluates the movement pattern 10' of the wearer 1 to infer non-presence at the hygiene station 103. At time point t_{d}, the badge 10 enters within range 11' of HST 11 and starts to intercept WS1. At time point tₗ, the badge 10 leaves the range 11' and no longer intercepts WS1. FIG. 5A also illustrates the location of the badge 10 at an intermediate time point tᵢ.

In some embodiments, the badge 10 starts to compile the movement pattern 10' at time point t_{d}. In other embodiments, the badge 10 is configured to continuously or intermittently determine the movement pattern of wearer and buffer the most recently determined movement pattern in internal memory (cf. 22 in FIG. 2A). For example, the badge 10 may buffer the movement pattern for the latest 10-60 seconds. Alternatively, the badge 10 may be configured to buffer raw data from the movement sensor 24 and determine the movement pattern as needed based on the raw data. By the buffering, more data is available for the evaluation in step 404 to thereby allow step 404 to also operate on the walking pattern of the wearer before WS1 is intercepted in step 401. It may be noted that even if the badge 10 buffers a movement pattern or raw data, the determination of the first association data in step 403 occurs upon interception of WS1 in step 401. Depending on implementation, the determination of first association data in step 403 may comprise retrieving and processing buffered data and/or receiving and processing the output signal of the movement sensor 24.

The movement pattern may be evaluated by step 404 in many different ways to infer presence or non-presence at the hygiene station 103. In FIG. 5A, step 404 may determine a first position at time point t_{d} and a second position at time point tₗ, and use the first and second positions as reference positions for evaluating the movement pattern 10', for example at one or more intermediate time points tᵢ. The first and second positions will locate the movement pattern 10' in relation to the range 11', thereby enabling quantitative evaluation of the movement pattern 10'. In one embodiment, the compliance criterion may infer presence at the hygiene station 103 if the movement pattern 10' exhibits a sufficient deviation from a connecting line 10" between the first and second positions. It is realized that the detection of tₗ may require HST 11 to transmit WS1 for a relatively long time period following activation (cf. TTL in FIG. 8). In one embodiment, the compliance criterion may infer presence/non-presence based on a time difference between the time points t_{d} and tₗ, i.e. time period during which the badge 10 receives WS1. For example, the compliance criterion may infer presence if the time difference exceeds a minimum time, and/or if the time difference is substantially equal to TTL.

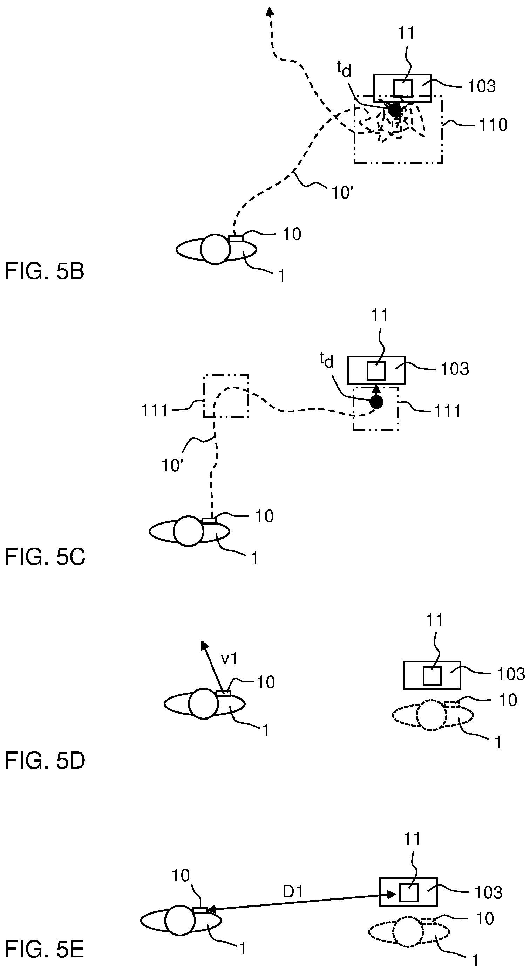

In some embodiments, step 404 evaluates the movement pattern 10' for detection of a sub-pattern that indicates that the wearer has stopped or a sub-pattern that is localized to a confined region with predefined dimensions, for example within an area of 1m by 1m. A sub-pattern is localized to a confined region if sub-pattern, for a predefined period of time, remains within the confined region. An example is shown in FIG. 5B, in which the wearer 1 moves to the hygiene station 103, performs a washing operation that activates the HST 11 at time point t_{d}, and moves away from the hygiene station 103. As seen, the movement pattern 10' will be localized to a confined area 110 and/or involve a stop of movement when the wearer 1 performs the washing operation. Thus, step 403 may apply a rule that infers presence if the movement pattern 10' comprises a stop or a localized sub-pattern 110 in relation to the time point t_{d}. FIG. 5B also illustrates that it may be beneficial for step 404 to not only evaluate the movement pattern 10' after time point t_{d}, but also before time point t_{d}, which is accomplished by the above-mentioned buffering.

In some embodiments, step 404 evaluates the movement pattern 10' for detection of characteristic changes in motion, such as deceleration, directional changes etc. An example is shown in FIG. 5C, in which the wearer 1 makes a sharp right turn 111 followed by a sharp left turn 111 to arrive at the washing station 103. Thus, the compliance criterion may infer presence if the movement pattern 10', before the activation of the HST 11, involves one or more directional changes 111 of sufficient magnitude. It is realized that the example in FIG. 5C requires the badge 10 to implement the above-mentioned buffering.

In some embodiments, the first association data is representative of a speed of the badge 10, which is determined based on the output signal of the movement sensor 24 (FIG. 2A). For example, it is well-known to estimate speed based on the output of one or more accelerometers. The compliance criterion may infer presence if the speed is below a speed threshold when the HST 11 is activated. FIG. 5D illustrates a use case in which the HST 11 is activated by another individual 1 (dashed lines), and the badge 10 of the wearer 1 (solid lines) determines and evaluates the speed v1 of the badge 1 to infer non-presence at the hygiene station 103.

In some embodiments, the first association data is representative of distance between the badge 10 and the HST 11. The distance parameter may be given by or derived from the signal strength (signal power) of WS1 at the receiver 23 of the badge 10. It is well-known in the art that the signal strength of a wireless signal depends on the distance travelled. In one example, the signal strength is obtained as an RSSI (Received Signal Strength Indicator). The compliance criterion may infer presence if the distance D1 is below a distance threshold and/or if a difference in distance D1 between two time points is below a speed threshold. It should be understood that the distance parameter may, but need not, be converted into actual distance. FIG. 5E illustrates a use case in which the HST 11 is activated by another individual 1 (dashed lines), and the badge 10 of the wearer 1 (solid lines) determines and evaluates the distance D1 to the HST 11 to infer non-presence at the hygiene station 103.

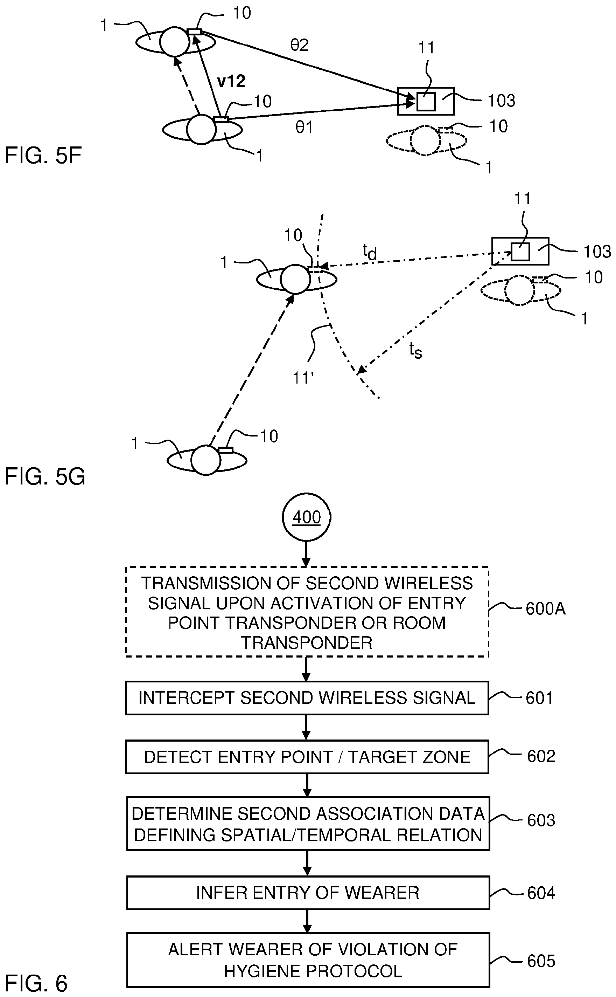

In some embodiments, the first association data is representative of a velocity vector which is representative of both speed (scalar) and direction of movement of the badge 10 in relation to HST 11. The velocity vector may be estimated based on the angle-of-arrival (AoA) of WS1 at the badge 10 at consecutive time points in combination with distance at these time points. FIG. 5F illustrates a use case in which the HST 11 is activated by another individual 1 (dashed lines), and the badge 10 of the wearer 1 (solid lines) detects and evaluates the velocity vector **v12** to infer non-presence at the hygiene station 103. In FIG. 5F, the AoA at two time points is represented by θ1 and θ2. The AoA may be determined for WS1 if the receiver 23 comprises plural antenna elements, as is well-known in the art. The distance may be determined as in FIG. 5E. It is realized that an approximate location of HST 11 may be determined based on distance and AoA at two or more time points. The compliance criterion may infer presence if the speed is below a speed threshold and/or the movement is generally directed towards the location of HST 11. For example, presence may be inferred if the direction of movement is within ±45° from a line extending between the badge 10 and HST 11.

FIG. 5G illustrates a scenario in which the first association data is representative of a time difference between initial receipt of WS1 and initial activation of HST 11. In FIG. 5G, the badge 10 is outside range 11' when HST 11 is activated at time point tₛ, and moves into range 11' at time point t_{d}. Assuming that WS1 includes the above-mentioned current timer value (FIG. 8), the time difference between tₛ and t_{d} may be directly inferred by extracting the current timer value from WS1. The compliance criterion may infer non-presence if the time difference is below a time threshold. In an alternative embodiment, HST 11 and the badge 10 operate in a common time frame, and HST 11 includes a time stamp of tₛ in WS1, and the badge 10 determines t_{d} and computes the difference between t_{d} and tₛ. However, the provision of a common time frame adds complexity to the monitoring system.

The compliance criterion may comprise a combination of rules, for example any of the rules implied by the description of FIGS 5A-5G, or any other rules, to increase the confidence of the assigned compliance status. Generally, as used herein, a step of "inferring presence" may involve directly inferring presence by one or more presence rules and/or indirectly inferring presence by one or more non-presence rules.

As noted in relation to step 404, the compliance criterion may also comprise one or more completion rules. One such completion rule may be that an estimated residence time of the wearer 1 at the hygiene station 103 exceeds a duration threshold. The residence time may be determined based on the output signal of the movement sensor 24 and/or WS1, for example as a function of the time period during which one or more presence rules indicate presence at the hygiene station 103. Another completion rule may be to detect a particular type of hygiene station or a particular hygiene station based on WS1 (cf. step 402 in FIG. 4). Yet another completion rule may be to detect two or more activated components at the hygiene station, or a particular order of activation of such components, assuming that the components are provided with HSTs 11 that include different TIDs in their respective WS1. In some embodiments, the duration threshold may be set in dependence of the hygiene station 103, for example to account for that fact that the complexity of the washing operation may vary between different hygiene stations or types of hygiene stations.

The method 400 in FIG. 4 may be implemented by a combination of badges 10 and HSTs 11. Such a monitoring system may be operated to collect statistics about the hygiene standard in a facility, for example the number/frequency of washing operations in total or per individual. The statistics may be obtained by downloading data from the respective badge 10, by wire or wirelessly. The monitoring system may also be operated to monitor the hygiene standard of specific individuals, for example based on a database associating badges 10 with wearers. The monitoring system may also be used to enforce adherence to a hygiene protocol, by the badge 10 evaluating settings of the status indicator in relation to a hygiene protocol and operating the feedback device 25 to alert the wearer whenever a violation is detected. Such a hygiene protocol may define a time schedule for performing washing operations, for example a maximum time between washing operations or a minimum frequency of washing operations.

FIG. 6 is a flow chart of an extension of the method 400, in which second wireless signals from EPTs 12 and/or RTs 13 are intercepted and processed by badges 10 to infer entry into a target zone that is subject to hygiene requirements, for example the room 101 in FIG. 1. Upon entry and depending on its current compliance status, the badge 10 may alert the wearer of a violation of a hygiene protocol. The method in FIG. 6 may be implemented by a combination of badges 10, HSTs 11, and at least one of EPTs 12 and RTs 13.

The method in FIG. 6 presumes a step 600A, which is performed by an EPT 12 or an RT 13 ("second transponder") and involves transmission of WS2. In step 601, the receiver 23 of the badge 10 intercepts WS2, if the receiver 23 is within range of the transmitter 23' of the second transponder 12/13. Subsequent steps 602-605 are performed by the processing system 20 of the badge 10. In step 602, the entry point or target zone is detected based on WS2. Step 602 may comprise determining that the intercepted signal is a WS2 and thus originates from a second transponder 12/13. Optionally, step 602 may also determine, based on the TID or supplemental data in WS2, that WS2 originates from a specific type of second transponder 12/13, for example an EPT 12 or an RT 13, or a specific type of target zone, or even a specific target zone. In step 603, second association data is determined based on WS2 and/or the output signal from the movement sensor 24 in the badge 10. The second association data defines a spatial and/or temporal relation between the badge 10 and the second transponder 12/13 that has transmitted WS2. The second association data from step 603 is then used by step 604 to infer if the wearer has entered the target zone of the second transponder 12/13. When step 604 infers entry, step 605 evaluates the current compliance status in relation to a hygiene protocol and alerts the wearer if a violation is detected, for example by activating the feedback device 25 in the badge 10. In one example, step 605 generates the alert if the wearer has a non-compliant status on entry into the target zone. In some embodiments, the hygiene protocol may differ between target zones or between types of target zone. Thus, the badge 10 may store different hygiene protocols, and retrieve and evaluate the hygiene protocol for the current target zone, as indicated by the TID in WS2. Clearly, the provision of TID enables the evaluation in step 605 to apply varying degrees of complexity and also enables the monitoring system to be expanded and updated with new functionality, for example new hygiene protocols, new categorizations of target zones, new types of transponders, etc.

In an alternative, step 605 may cause an external feedback device, for example mounted in the target zone, to alert the wearer of the violation. For example, the badge 10 may broadcast a violation signal that, upon receipt, causes the external feedback unit to generate a feedback signal to alert the wearer.

The second association data may comprise any of the spatial and/or temporal parameters described above for the first association data, including the parameters disclosed with reference to FIGS 5A-5G. The evaluation in step 604 may apply one or more rules indicative of non-entry and/or one or more rules indicative of entry, to infer whether the wearer has entered the target zone. The evaluation may differ between EPTs 12 and RTs 13, given their different placements. A rule indicative of entry through a door provided with an EPT 12 may detect that the badge 10 moves, stops and then starts to move again. Further rules indicative of entry and applicable to EPTs 12 may comprise that there is a significant angular change of direction of movement (by the wearer turning into the room 101 from the corridor 100), that the movement pattern sufficiently matches a predefined movement pattern, that the time difference in FIG. 5G is below a difference threshold, that the distance decreases and then increases again, etc. Rules indicative of non-entry, applicable to EPTs 12, may comprise that the speed is above a speed threshold during an evaluation time period from step 601, that the distance is above a distance threshold during the evaluation time period, that the direction of movement is generally invariant during the evaluation time period, etc. Several rules may be combined and evaluated in time synchronization, for example requiring a temporal correlation between changes in distance and direction, etc.

A rule indicative of entry, applicable to RTs 13, may comprise that the distance decreases while the badge 10 is moving. The distance may be determined based on WS2, by analogy with FIG. 5E, and that the badge 10 is moving may be detected from the output signal of the movement sensor 24 in the badge 10. Rules indicative of non-entry, applicable to RTs 13, may comprise that the speed is above a speed threshold during an evaluation time period from step 601, that the distance is above a distance threshold during the evaluation time period, etc.

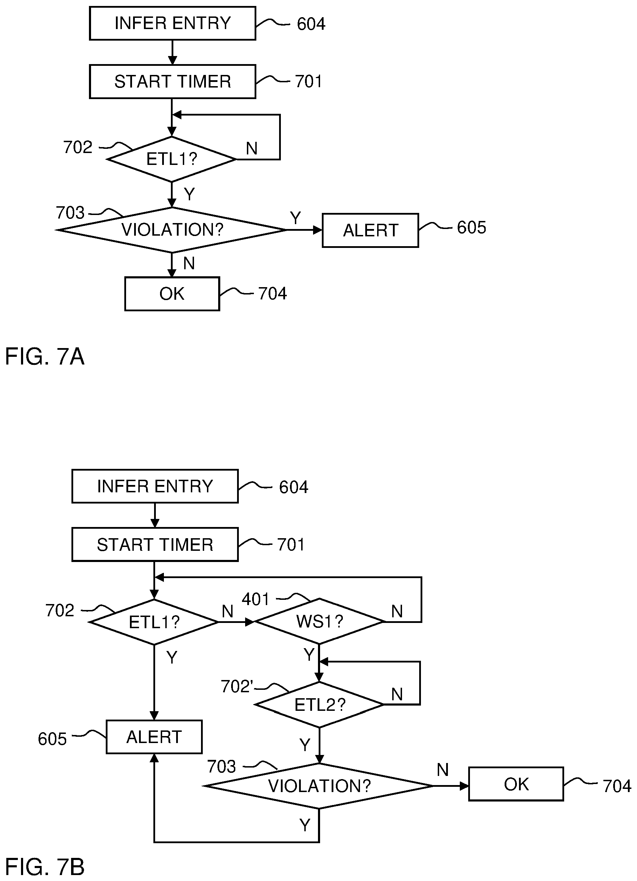

The example method in FIG. 6 presumes that hygiene stations 103 are located outside the target zone 101 and that the method 400 is performed in advance of entry into the target zone 101. FIG. 7A is a flow chart of an alternative method, which is performed by a badge 10 and allows the hygiene station 103 to be placed inside the target zone 101. When the method infers entry of a wearer, for example based on steps 601-604 as described for FIG. 6, step 701 starts an entry timer and waits for expiry of an entry time limit, ETL1. When step 702 detects that ETL1 is reached, step 703 checks if the hygiene protocol is violated. If a violation is detected, step 605 alerts the wearer, otherwise the method is completed. The provision of ETL1, gives the wearer time to perform a washing operation at the hygiene station in the target zone, which may be detected by the badge 10 in accordance with the method 400 in FIG. 4.

FIG. 7B is a flow chart of an alternative to the method in FIG. 7A. The method in FIG. 7B will give the wearer a first time period, represented by ETL1, to activate HST 11 at the hygiene station, and a second time period, represented by ETL2, to complete the washing operation. Specifically, while waiting for expiry of ETL1, the method waits for interception of WS1 in accordance with step 401. If WS1 is not received within ETL1, step 605 alerts the wearer. If WS1 is received, the method proceeds to wait for expiry of ETL2, which is a second entry time limit. When step 702' detects that ETL2 is reached, step 703 checks if the hygiene protocol is violated. If a violation is detected, step 605 alerts the wearer, otherwise the method is completed.

The technique of inferring entry of a wearer into a target zone as described hereinabove may also be used independent of any preceding hygiene operation by the wearer. In some embodiments, the badge 10 is configured to alert the wearer whenever the wearer enters a target zone that is associated with a hygiene protocol. Such an alert will serve as a reminder for the wearer to consider the hygiene protocol and may cause the wearer to leave the target zone, check the current hygiene protocol, go to a hygiene station in the target zone and perform a washing operation, etc. FIG. 9 is a flowchart of an corresponding method, which may serve to enhance hygiene compliance by the wearer of the badge. When the method infers entry of the wearer, for example based on steps 601-604 as described for FIG. 6, step 605' alerts the wearer to consider a hygiene protocol. In some embodiments, step 605' alerts the wearer only if the target zone is of a specific type, if determined by step 602. In contrast to the example method in FIG. 6, the method in FIG. 9 generates the alert independent of the current compliance status of the wearer of the badge 10.

Although the foregoing examples are given in the context of a healthcare facility, the disclosure is equally applicable for hygiene compliance monitoring in other environments, including but not limited to facilities for food processing, preparation or distribution.

## Claims

1. A wearable device for monitoring hygiene compliance by a wearer, said wearable device comprising a receiver (23) for wireless signals, wherein the wearable device is configured to, when the receiver (23) intercepts a first wireless signal transmitted from a first transponder (11) when activated by a user of a hygiene station (103):
detect the hygiene station (103) based on the first wireless signal;
evaluate a compliance criterion to infer presence of the wearer at the hygiene station (103); and
set a hygiene compliance status of the wearer to compliant if the compliance criterion is fulfilled, **characterized in that**
the wearable device comprises a movement sensor (24);
the wearable device is configured to determine first association data as a function of an output signal of the movement sensor (24), wherein the first association data is at least representative of a movement pattern (10') of the wearable device corresponding to a walking pattern of the wearer, and
the wearable device is configured to evaluate the compliance criterion as a function of the movement pattern (10') to infer the presence of the wearer at the hygiene station (103).

2. The wearable device of claim 1, which is further configured to determine the first association data as a function of the first wireless signal.

3. The wearable device of any preceding claim, wherein the wearable device is configured to infer presence by detecting that the movement pattern (10') comprises a sub-pattern that represents a stop in the walking pattern of the wearer or a sub-pattern that is localized to a confined region.

4. The wearable device of any preceding claim, which is configured to infer presence by detecting that the movement pattern (10') comprises a characteristic change in motion.

5. The wearable device of any preceding claim, which is configured to continuously or intermittently buffer data corresponding to the movement pattern in a memory (22), wherein the wearable device is configured to determine the first association data by retrieving at least part of the data buffered in the memory (22).

6. The wearable device of any preceding claim, wherein the movement pattern (10') at least partly represents the walking pattern of the wearer before the receiver (23) intercepts the first wireless signal.

7. The wearable device of any preceding claim, which is configured to, based on the movement pattern (10'), determine a first position when the receiver (23) first intercepts the first wireless signal, and determine a second position when the first wireless signal subsequently disappears at the receiver (23), wherein the wearable device is configured to evaluate the movement pattern (10') in relation to the first and second positions to infer presence of the wearer at the hygiene station (103).

8. The wearable device of any preceding claim, which is further configured to determine, based on the first wireless signal and/or the output signal of the movement sensor (24), a residence time of the wearer at the hygiene station (103), wherein the compliance criterion further comprises comparing the residence time to a duration threshold.

9. The wearable device of any preceding claim, wherein the first association data is further representative of a distance to the first transponder (11), and wherein the wearable device is configured to infer presence by detecting that the distance is below a distance threshold.

10. The wearable device of any preceding claim, wherein the first association data is further representative of a speed of the wearable device, and wherein the wearable device is configured to infer presence by detecting that the speed is below a speed threshold.

11. The wearable device of any preceding claim, wherein the first association data is further representative of a direction of movement of the wearable device in relation to the first transponder (11), and wherein the wearable device is configured to infer presence by comparing the direction to a location of the hygiene station (103).

12. The wearable device of any preceding claim, wherein the first association data is further representative of a time difference between initial receipt of the first wireless signal by the receiver (23) and initial activation of the first transponder (11), and wherein the wearable device is configured to detect presence by detecting that the time difference is below a time threshold.

13. The wearable device of any preceding claim, which is configured to, when the receiver (23) intercepts a second wireless signal transmitted from a second transponder (12) located at a target zone (101):
detect the target zone (101) based on the second wireless signal;
determine second association data as a function of at least one of the second wireless signal or the output signal of the movement sensor (24), the second association data defining a spatial and/or temporal relation between the wearable device and the second transponder (12); and
infer entry of the wearer into the target zone (101) as a function of the second association data.

14. A system for monitoring hygiene compliance, comprising:
a first transponder (11), which is configured to be installed at a hygiene station (103) for selective activation by users of the hygiene station (103), the first transponder (11) being configured to, when activated, to transmit a first wireless signal, and
a wearable device (10) in accordance with any one of claims 1-13.

15. A method of operating a wearable device for monitoring hygiene compliance by a wearer, said method comprising, upon intercepting (401) a first wireless signal transmitted from a first transponder when activated by a user of a hygiene station:
detecting (402) the hygiene station based on the first wireless signal;
evaluating (404) a compliance criterion to infer presence of the wearer at the hygiene station; and
setting (405) a hygiene compliance status of the wearer to compliant if the compliance criterion is fulfilled, **characterized by**
determining (403) first association data as a function of an output signal of a movement sensor in the wearable device, said first association data being at least representative of a movement pattern of the wearable device corresponding to a walking pattern of the wearer; and
the compliance criterion being evaluated as a function of the movement pattern (10') to infer the presence of the wearer at the hygiene station (103).

## Patentansprüche

1. Tragbare Vorrichtung zum Überwachen der Hygienekonformität eines Trägers, wobei die tragbare Vorrichtung einen Empfänger (23) für drahtlose Signale umfasst, wobei, wenn der Empfänger (23) ein erstes drahtloses Signal abfängt, das von einem ersten Transponder (11) gesendet wird, wenn durch einen Benutzer einer Hygienestation (103) aktiviert, die tragbare Vorrichtung ausgelegt ist zum:
Detektieren der Hygienestation (103) basierend auf dem ersten drahtlosen Signal;
Evaluieren eines Konformitätskriteriums, um auf die Anwesenheit des Trägers an der Hygienestation (103) zu schließen; und
Setzen eines Hygienekonformitätsstatus des Trägers als "konform", wenn das Konformitätskriterium erfüllt ist, **dadurch gekennzeichnet, dass**
die tragbare Vorrichtung einen Bewegungssensor (24) umfasst;
die tragbare Vorrichtung ausgelegt ist zum Bestimmen erster Assoziationsdaten als eine Funktion eines Ausgangssignals des Bewegungssensors (24), wobei die ersten Assoziationsdaten mindestens repräsentativ für ein Bewegungsmuster (10') der tragbaren Vorrichtung sind, das einem Gehmuster des Trägers entspricht, und
die tragbare Vorrichtung ausgelegt ist zum Evaluieren des Konformitätskriteriums als eine Funktion des Bewegungsmusters (10'), um auf die Anwesenheit des Trägers an der Hygienestation (103) zu schließen.

2. Tragbare Vorrichtung nach Anspruch 1, die ferner ausgelegt ist zum Bestimmen der ersten Assoziationsdaten als eine Funktion des ersten drahtlosen Signals.

3. Tragbare Vorrichtung nach einem der vorhergehenden Ansprüche, wobei die tragbare Vorrichtung ausgelegt ist zum Schließen auf eine Anwesenheit durch Detektieren, dass das Bewegungsmuster (10') ein Untermuster, das eine Unterbrechung im Gehmuster des Trägers repräsentiert, oder ein Untermuster, das in einem begrenzten Gebiet lokalisiert ist, umfasst.

4. Tragbare Vorrichtung nach einem der vorhergehenden Ansprüche, die ausgelegt ist zum Schließen auf eine Anwesenheit durch Detektieren, dass das Bewegungsmuster (10') eine charakteristische Bewegungsänderung umfasst.

5. Tragbare Vorrichtung nach einem der vorhergehenden Ansprüche, die ausgelegt ist zum kontinuierlichen oder intermittierenden Puffern von Daten entsprechend dem Bewegungsmuster in einem Speicher (22), wobei die tragbare Vorrichtung ausgelegt ist zum Bestimmen der ersten Assoziationsdaten durch Abrufen mindestens eines Teils der in dem Speicher (22) gepufferten Daten.

6. Tragbare Vorrichtung nach einem der vorhergehenden Ansprüche, wobei das Bewegungsmuster (10') zumindest teilweise das Gehmuster des Trägers repräsentiert, bevor der Empfänger (23) das erste drahtlose Signal abfängt.

7. Tragbare Vorrichtung nach einem der vorhergehenden Ansprüche, die ausgelegt ist zum Bestimmen, basierend auf dem Bewegungsmuster (10'), einer ersten Position, wenn der Empfänger (23) zuerst das erste drahtlose Signal abfängt, und Bestimmen einer zweiten Position, wenn das erste drahtlose Signal anschließend an dem Empfänger (23) verschwindet, wobei die tragbare Vorrichtung ausgelegt ist zum Evaluieren des Bewegungsmusters (10') in Bezug auf die erste und die zweite Position, um auf eine Anwesenheit des Trägers an der Hygienestation (103) zu schließen.

8. Tragbare Vorrichtung nach einem der vorhergehenden Ansprüche, die ferner ausgelegt ist zum Bestimmen, basierend auf dem ersten drahtlosen Signal und/oder dem Ausgangssignal des Bewegungssensors (24), einer Verweilzeit des Trägers an der Hygienestation (103), wobei das Konformitätskriterium ferner das Vergleichen der Verweilzeit mit einer Dauerschwelle umfasst.

9. Tragbare Vorrichtung nach einem der vorhergehenden Ansprüche, wobei die ersten Assoziationsdaten ferner repräsentativ für einen Abstand zu dem ersten Transponder (11) sind und wobei die tragbare Vorrichtung ausgelegt ist zum Schließen auf eine Anwesenheit durch Detektieren, dass der Abstand unter einer Abstandsschwelle liegt.

10. Tragbare Vorrichtung nach einem der vorhergehenden Ansprüche, wobei die ersten Assoziationsdaten ferner repräsentativ für eine Geschwindigkeit der tragbaren Vorrichtung sind und wobei die tragbare Vorrichtung ausgelegt ist zum Schließen auf eine Anwesenheit durch Detektieren, dass die Geschwindigkeit unter einer Geschwindigkeitsschwelle liegt.

11. Tragbare Vorrichtung nach einem der vorhergehenden Ansprüche, wobei die ersten Assoziationsdaten ferner repräsentativ für eine Bewegungsrichtung der tragbaren Vorrichtung in Bezug auf den ersten Transponder (11) sind und wobei die tragbare Vorrichtung ausgelegt ist zum Schließen auf eine Anwesenheit durch Vergleichen der Richtung mit einem Ort der Hygienestation (103).

12. Tragbare Vorrichtung nach einem der vorhergehenden Ansprüche, wobei die ersten Assoziationsdaten ferner repräsentativ für eine Zeitdifferenz zwischen dem anfänglichen Empfang des ersten drahtlosen Signals durch den Empfänger (23) und der anfänglichen Aktivierung des ersten Transponders (11) sind und wobei die tragbare Vorrichtung ausgelegt ist zum Detektieren einer Anwesenheit durch Detektieren, dass die Zeitdifferenz unter einer Zeitschwelle liegt.

13. Tragbare Vorrichtung nach einem der vorhergehenden Ansprüche, die, wenn der Empfänger (23) ein zweites drahtloses Signal abfängt, das von einem zweiten Transponder (12) gesendet wird, der sich in einer Zielzone (101) befindet, ausgelegt ist zum:
Detektieren der Zielzone (101) basierend auf dem zweiten drahtlosen Signal;
Bestimmen zweiter Assoziationsdaten als eine Funktion des zweiten drahtlosen Signals und/oder des Ausgangssignals des Bewegungssensors (24), wobei die zweiten Assoziationsdaten eine räumliche und/oder zeitliche Beziehung zwischen der tragbaren Vorrichtung und dem zweiten Transponder (12) definieren; und
Schließen auf einen Eintritt des Trägers in die Zielzone (101) als Funktion der zweiten Assoziationsdaten.

14. System zur Überwachung der Hygienekonformität, das Folgendes umfasst:
einen ersten Transponder (11), der dazu ausgelegt ist, an einer Hygienestation (103) zur gezielten Aktivierung durch Benutzer der Hygienestation (103) installiert zu werden, wobei der erste Transponder (11), wenn aktiviert, ausgelegt ist zum Senden eines ersten drahtlosen Signals, und
eine tragbare Vorrichtung (10) nach einem der Ansprüche 1-13.

15. Verfahren zum Betreiben einer tragbaren Vorrichtung zum Überwachen der Hygienekonformität eines Trägers, wobei das Verfahren beim Abfangen (401) eines ersten drahtlosen Signals, das von einem ersten Transponder gesendet wird, wenn durch einen Benutzer einer Hygienestation aktiviert, Folgendes umfasst:
Detektieren (402) der Hygienestation basierend auf dem ersten drahtlosen Signal;
Evaluieren (404) eines Konformitätskriteriums, um auf die Anwesenheit des Trägers an der Hygienestation zu schließen; und
Setzen (405) eines Hygienekonformitätsstatus des Trägers auf "konform", wenn das Konformitätskriterium erfüllt ist, **gekennzeichnet durch**
Bestimmen (403) erster Assoziationsdaten als eine Funktion eines Ausgangssignals eines Bewegungssensors in der tragbaren Vorrichtung, wobei die ersten Assoziationsdaten mindestens repräsentativ für ein Bewegungsmuster der tragbaren Vorrichtung sind, das einem Gehmuster des Trägers entspricht; und
wobei das Konformitätskriterium in Abhängigkeit vom Bewegungsmuster (10') evaluiert wird, um auf die Anwesenheit des Trägers an der Hygienestation (103) zu schließen.

## Revendications

1. Dispositif portable permettant de surveiller la conformité à l'hygiène d'un porteur, ledit dispositif portable comprenant un récepteur (23) pour signaux sans fil, le dispositif portable étant configuré pour, lorsque le récepteur (23) intercepte un premier signal sans fil transmis par un premier transpondeur (11) lorsque celui-ci est activé par un utilisateur d'un poste d'hygiène (103) :
détecter le poste d'hygiène (103) en fonction du premier signal sans fil ;
évaluer un critère de conformité pour déduire la présence du porteur au poste d'hygiène (103) ; et
définir un statut de conformité à l'hygiène du porteur comme conforme si le critère de conformité est rempli, **caractérisé en ce que**
le dispositif portable comprend un capteur de mouvement (24) ;
le dispositif portable est configuré pour déterminer des premières données d'association en fonction d'un signal de sortie du capteur de mouvement (24), lesdites premières données d'association représentant au moins un schéma de déplacement (10') du dispositif portable correspondant à un schéma de marche du porteur, et
le dispositif portable est configuré pour évaluer le critère de conformité en fonction du schéma de déplacement (10') pour déduire la présence du porteur au poste d'hygiène (103).

2. Dispositif portable selon la revendication 1, qui est en outre configuré pour déterminer les premières données d'association en fonction du premier signal sans fil.

3. Dispositif portable selon l'une quelconque des revendications précédentes, dans lequel le dispositif portable est configuré pour déduire la présence en détectant que le schéma de déplacement (10') comprend un sous-schéma qui représente un arrêt dans le schéma de marche du porteur ou un sous-schéma qui est localisé dans une région confinée.

4. Dispositif portable selon l'une quelconque des revendications précédentes, qui est configuré pour déduire la présence en détectant que le schéma de déplacement (10') comprend un changement de mouvement caractéristique.

5. Dispositif portable selon l'une quelconque des revendications précédentes, qui est configuré pour mettre en mémoire tampon, de manière continue ou intermittente, dans une mémoire (22), des données correspondant au schéma de déplacement, le dispositif portable étant configuré pour déterminer les premières données d'association en récupérant au moins une partie des données mises en mémoire tampon dans la mémoire (22).

6. Dispositif portable selon l'une quelconque des revendications précédentes, dans lequel le schéma de déplacement (10') représente au moins partiellement le schéma de marche du porteur avant que le récepteur (23) intercepte le premier signal sans fil.

7. Dispositif portable selon l'une quelconque des revendications précédentes, qui est configuré pour, sur la base du schéma de déplacement (10'), déterminer une première position lorsque le récepteur (23) intercepte pour la première fois le premier signal sans fil, et déterminer une deuxième position lorsque le première signal sans fil disparaît ensuite au niveau du récepteur (23), le dispositif portable étant configuré pour évaluer le schéma de déplacement (10') en relation avec les première et deuxième positions pour déduire la présence de l'utilisateur au poste d'hygiène (103).

8. Dispositif portable selon l'une quelconque des revendications précédentes, qui est en outre configuré pour déterminer, sur la base du premier signal sans fil et/ou du signal de sortie du capteur de mouvement (24), un temps de résidence du porteur au poste d'hygiène (103), le critère de conformité comprenant en outre la comparaison du temps de résidence à un seuil de durée.

9. Dispositif portable selon l'une quelconque des revendications précédentes, dans lequel les premières données d'association représentent en outre une distance par rapport au premier transpondeur (11), et dans lequel le dispositif portable est configuré pour déduire la présence en détectant que la distance est inférieure à un seuil de distance.

10. Dispositif portable selon l'une quelconque des revendications précédentes, dans lequel les premières données d'association représentent en outre une vitesse du dispositif portable, et dans lequel le dispositif portable est configuré pour déduire la présence en détectant que la vitesse est inférieure à un seuil de vitesse.

11. Dispositif portable selon l'une quelconque des revendications précédentes, dans lequel les premières données d'association représentent en outre une direction de déplacement du dispositif portable par rapport au premier transpondeur (11), et dans lequel le dispositif portable est configuré pour déduire la présence en comparant la direction à un emplacement du poste d'hygiène (103).

12. Dispositif portable selon l'une quelconque des revendications précédentes, dans lequel les premières données d'association représentent en outre une différence de temps entre la réception initiale du premier signal sans fil par le récepteur (23) et l'activation initiale du premier transpondeur (11), et dans lequel le dispositif portable est configuré pour détecter la présence en détectant que la différence de temps est inférieure à un seuil temporel.

13. Dispositif portable selon l'une quelconque des revendications précédentes, qui est configuré pour, lorsque le récepteur (23) intercepte un deuxième signal sans fil transmis par un deuxième transpondeur (12) situé au niveau d'une zone cible (101) :
détecter la zone cible (101) sur la base du deuxième signal sans fil ;
déterminer des deuxièmes données d'association en fonction d'au moins l'un du deuxième signal sans fil et du signal de sortie du capteur de mouvement (24), les deuxièmes données d'association définissant une relation spatiale et/ou temporelle entre le dispositif portable et le deuxième transpondeur (12) ; et
déduire l'entrée du porteur dans la zone cible (101) en fonction des deuxièmes données d'association.

14. Système de surveillance de conformité à l'hygiène, comprenant :
un premier transpondeur (11), qui est configuré pour être installé à un poste d'hygiène (103) pour une activation sélective par des utilisateurs du poste d'hygiène (103), le premier transpondeur (11) étant configuré pour, lorsqu'il est activé, transmettre un premier signal sans fil, et
un dispositif portable (10) selon l'une quelconque des revendications 1 à 13.

15. Procédé de fonctionnement d'un dispositif portable permettant de surveiller la conformité à l'hygiène d'un porteur, ledit procédé comprenant, lors de l'interception (401) d'un premier signal sans fil transmis par un premier transpondeur lorsqu'il est activé par un utilisateur d'un poste d'hygiène :
la détection (402) du poste d'hygiène sur la base du premier signal sans fil ;
l'évaluation (404) d'un critère de conformité pour déduire la présence du porteur au poste d'hygiène ; et
la définition (405) d'un statut de conformité à l'hygiène du porteur comme conforme si le critère de conformité est rempli, **caractérisé par**
la détermination (403) de premières données d'association en fonction d'un signal de sortie du capteur de mouvement dans le dispositif portable, lesdites premières données d'association représentant au moins un schéma de déplacement du dispositif portable correspondant à un schéma de marche du porteur ; et
le critère de conformité étant évalué en fonction du schéma de déplacement (10') pour déduire la présence du porteur au poste d'hygiène (103).
